**Europäisches Patentamt**

**European Patent Office**

**Office européen des brevets**

(11) Publication number : **0 288 556 B1**

(12) # EUROPEAN PATENT SPECIFICATION

(45) Date of publication of patent specification :
13.05.92 Bulletin 92/20

(51) Int. Cl.⁵ : **G21G 4/08**

(21) Application number : **87907875.6**

(22) Date of filing : **30.10.87**

(86) International application number :
**PCT/US87/02814**

(87) International publication number :
**WO 88/03697 19.05.88 Gazette 88/11**

(54) RHENIUM GENERATOR SYSTEM AND METHOD FOR ITS PREPARATION AND USE.

(30) Priority : **10.11.86 US 929644**

(43) Date of publication of application :
**02.11.88 Bulletin 88/44**

(45) Publication of the grant of the patent :
**13.05.92 Bulletin 92/20**

(84) Designated Contracting States :
**AT BE CH DE FR GB IT LI LU NL SE**

(56) References cited :
**Chemical Abstracts, volume 66,1967, (Columbus, Ohio, US), V.I. Plotnikov et al.:
"Separation of small amounts of rhenium andtungsten with zirconium hydroxide", see abstract no. 16227c,
Chemical Abstracts, volume 83, 1975, (Columbus, Ohio, US), K.V. Malyshev et al.:
"Rhenium-188 generator based on hydrated-zirconium oxide", see page 421, abstract 49831z,
Chemical Abstracts, volume 69, 1968, (Columbus, Ohio, US), E. Klockow: "Carrier-free separation of rhenium isotopes from adeuteronirradiated tungsten matrix", see page 6827, abstract 73129h,**

(56) References cited :
**Chemical Abstracts, volume 105, 1986, (Columbus, Ohio, US), N. Botros et al.:
"Comparative studies on the development of atungsten-188/rhenium-188 generator", see page 490, abstract, 160459w,**

(73) Proprietor : **NEORX CORPORATION
410 West Harrison Street
Seattle Washington 98119 (US)**
Proprietor : **THE CURATORS OF THE UNIVERSITY OF MISSOURI
227 University Hall
Columbia Missouri 65211 (US)**

(72) Inventor : **EHRHARDT, Gary, J.
4238 Country Hill
Columbia, MO 65203 (US)**
Inventor : **VANDERHEYDEN, Jean-Luc, E.
2418 West Lynn
Seattle, WA 98199 (US)**
Inventor : **SU, Fu-Min
3254 N.E. 100th Street
Seattle, WA 98125 (US)**

(74) Representative : **Cookson, Barbara Elizabeth et al
WITHERS & ROGERS 4 Dyer's Buildings
Holborn
London EC1N 2JT (GB)**

EP 0 288 556 B1

## Description

This invention relates to the production of radionuclides useful for therapeutic and diagnostic medical applications, particularly to radionuclide generator systems, and more particularly to tungsten-188/rhenium-188 generators.

Radionuclides, meaning atomic species that exhibit radioactivity, are useful for diagnostic and therapeutic techniques such as tumor imaging and radiotherapy of tumors. Such techniques have increased the demand for available supplies of carrier-free radionuclides having reasonable half-lives such as Technetium-99m (Tc-99m, half-life 6.02 hours) used for diagnostic purposes. One method of obtaining such radionuclides is via extraction of a "daughter" radionuclide which is a decay product of a long-lived ("parent") radionuclide. For example, Tc-99m is the daughter radionuclide of molybdenum-99 (Mo-99, half-life 66.02 hrs).

Devices known as generators are commercially available to provide separation of a daughter radionuclide from its parent radionuclide to provide a supply of the relatively short-lived daughter isotope. The parent and daughter radionuclides may be separated using chromatographic, solvent extraction, or sublimation generators. The chromatographic generators, due to their simplicity and compact nature, are more convenient to use in hospitals and other institutions where radionuclides are used for diagnosis and therapy. For use in such generators, the parent radionuclide should have a sufficiently long half-life to provide enough time for transit and storage prior to commencing the extraction procedure.

Chromatographic generators, such as those used to produce Tc-99m from Mo-99, typically contain insolubilized parent radionuclide absorbed onto a bed or column of material such as aluminum oxide ("alumina") for which the daughter radionuclide has relatively little affinity. The daughter radionuclide, which forms from decay of the parent, is then periodically eluted from the column, for example using physiological saline. Typically, the daughter radionuclide product will be of high specific activity and is referred to as "carrier free" since it is produced by beta decay of a parent radionuclide, and the product is relatively free of stable isotopes of the daughter radionuclide.

Until recently, prior chromatographic generators were only able to provide high specific activity product radionuclides at relatively low concentrations from low specific activity (n, $\gamma$) parent radionuclides such as Mo-99 due to the necessity of using large quantities of alumina and eluting solution to obtain the daughter radionuclide. As a result, fission-produced parent radionuclides have been preferred for producing radionuclides such as Tc-99m. Unfortunately, fissionproduced radionuclides require complex facilities and safety precautions that entail high costs relative to the amount of daughter radionuclide produced.

Recently, a chromatographic Mo-99/Tc-99m generator has been developed that employs a matrix composed of zirconium molybdate containing Mo-99. Evans et al., U.S. Patent No. 4,280,053. This matrix is said to be essentially non-elutable, and to allow the Tc-99m produced in the matrix to diffuse through and from the matrix during elution.

Another radionuclide which shows promise for therapeutic and diagnostic applications is rhenium-188 (Re-188), a decay product of tungsten-188 (W-188), a low specific activity isotope produced from naturally occurring tungsten (W-186).

Although the chemical properties of rhenium are not as well known as those of technetium, certain of its properties suggest it may be suited for use in radiotherapeutic applications, for example, as a label for conjugation to monoclonal antibodies for targeting to tumors. Re-188 (half-life 16.98 hours) has a longer half-life than Tc-99m (6.02 hours), possesses a strong particulate emission (beta energy of 2.12 MeV) (in contrast, Tc-99m has no particulate emission), and has an imageable gamma emission (15%, 155 keV) ideal for current gamma camera imaging of tumors. W-188 radionuclide is derived from either natural tungsten (W-186) or, preferably, from a target tungsten material enriched in W-186 by double neutron capture using a high-flux reactor. The nuclear properties of this isotopic system are as follows:

| W-186 (stable) | (n, λ) ⟶ | W-187 (23.9 hr) β - Re-187 (stable) | (n, λ) ⟶ | W-188 (69.4d) β - Re-188 (16.98 hr) β - Os-188 (stable) |
| --- | --- | --- | --- | --- |

Previous tungsten/rhenium generators have consisted of small, alumina columns with relatively small amounts of tungsten targets adsorbed on the columns and, thus, low rhenium yields in the microcurie ($\mu$Ci) range. To increase the amount of rhenium obtainable from such columns (i.e., in the millicurie range, mCi), larger column masses are necessary in order to contain larger amounts of target tungsten. These larger columns, in turn, require increased eluting volumes.

In addition, prior W-188/Re-188 generators using alumina columns have provided poor yields of Re-188 and unacceptable level of release, or "breakthrough" of W-188 from the column due primarily to the necessity of adsorbing large (0.5 - 2.0 grams) amounts of target tungsten (primarily as W-186) onto the alumina column. A W-188/Re-188 generator system incorporating larger amounts of target tungsten to produce high yields (millicuries) of carrier-free Re-188 in small volumes (milliliters) without significant W-188 contamination would be useful for therapeutic and diagnostic applications.

Accordingly, the present invention provides a process for preparing a tungsten-188/rhenium-188 generator employing a tungstate generator matrix containing low specific activity W-188 produced by irradiating W-186. The matrix may be placed in an elutable container, such as a column, for harvesting substantially carrier-free, high specific activity Re-188. The process for preparing the generator matrix, composed of tungsten trioxide and zirconium, includes dissolving irradiated tungsten trioxide in a heated basic solution and adding this solution to an acidic zirconium-containing solution to form an acidic slurry in which zirconyl tungstate precipitate forms. An alternative process for preparing the generator of the present invention includes the additional step of adjusting the pH of the precipitate from about 2.8 to about 6.0, preferably about 4.3. Also, the slurry may be neutralized using a basic solution. W-188 radionuclide produced by irradiation is contained within the precipitate used to form the generator matrix. Rhenium-188 may be recovered by eluting the generator matrix with a saline solution, and the eluate may be further purified using an alumina or zirconium oxide substrate.

Chemical Abstracts, volume 83, 1975, (Columbus, Ohio, US), K. V. Malyshev et al. "Rhenium-188 generator based on hydrated zirconium oxide", see page 421, abstract 49831Z, and Radiokhimiya 1975, 17(2), 249-51, disclose a rhenium-188 generator using a zirconium oxyde precipitate as basic matrix and eluting the formed rhenium-188 from the generator with distilled water.

Chemical Abstracts, voume 69, 1968, Columbus, Ohio, US), E. Klockow: "Carrier-free separation of rhenium isotopes from a deuteron-irradiated tungsten-matrix", see page 6827, abstract 73129h, and J. Radioanal. Chem. 1968, 1⁻(4), 325-9, disclose a rhenium-isotopes generator also using a zirconium oxide precipitate as basic matrix. The formed rhenium-isotopes are separated by using 2% ammonium nitrate solution.

The present invention provides a process for preparing a radionuclide generator useful for producing Re-188, comprising the steps of:

(a) reacting a basic tungstate solution comprising W-188 with an acidic solution comprising zirconyl ion to form a zirconyl tungstate precipitate containing W-188 thereby forming a final gel or slurry which remains acidic,

(b) optionally adjusting the pH of said precipitate from about 2.8 to about 6; and,

(c) disposing said precipitate in an elutable container.

The present invention provides a process for preparing a W-188/Re-188 generator in the form of a substantially insoluble matrix containing W-188 which is permeable to diffusion of Re-188 in the form of the perrhenate ion ($ReO_4^-$). The matrix is preferably composed of a W-186 tungsten-containing material, such as a tungstate compound, which is irradiated to form relatively low specific activity W-188. The resulting parent radionuclide material is then dissolved in a heated basic solution, which is then combined with an acidic zirconium solution to precipitate the zirconyl tungstate matrix. The matrix, which contains the parent radionuclide W-188, may then be placed in a container for harvesting the desired high specific activity daughter radionuclide Re-188 by elution. Details of the preparation of the generator matrix and its use are described below.

The substantially insoluble precipitate containing the parent radionuclide, W-188, is prepared for use in forming a permeable matrix which allows a high rate of diffusion of the daughter radionuclide ion ($ReO_4^-$) generated as a decay product of the parent. Through this procedure, the Re-188 is produced in high yield and at a relatively high specific activity using simple elution methods, without requiring large beds or columns and thus the large elution volumes of prior method. Tungsten (W) in the form of a tungstate compound, derived from tungsten-186 trioxide ($WO_3$) or tungsten metal, when suitably combined with a soluble salt of a metallic cation, such as zirconyl ($ZrO^{+2}$), to form a precipitate, provides a matrix that is highly insoluble in solutions such as physiological saline that is commonly used to elute chromatogaphic generators.

The tungsten starting material is irradiated at high neutron flux levels using, for example, a 10 megawatt nuclear reactor to produce W-188, the parent radionuclide. Since tungsten trioxide is unstable in acid, to form the tungstate precipitate, the irradiated tungsten trioxide is preferably initially dissolved in a basic solution with a hydroxyl concentration ([OH]) of from 2 to 10 moles/liter. The base may be sodium hydroxide, potassium hydroxide, ammonium hydroxide or a similar source of hydroxil ion. Preferably, the basic solution has been heated to a temperature in the range of 50°C to 60°C to dissolve the tungsten trioxide. A more preferred heating range is 70°C to 90°C. The resulting solution may then be cooled to room temperature and combined with an acidic aqueous solution, such as hydrochloric acid (HCl), nitric acid (HNO$_3$) or sulfuric acid (H$_2$SO$_4$) with a hydrogen ion concentration ([H$^+$]) of from 1 to 6 moles/liter and preferably from 4 to 5 moles/liter, and containing zirconyl ion. It was found that when the basic and acid solutions were combined at temperatures in the range of from approximately 5°C to 80°C, for each solution, the precipitation of zirconyl tungstate still occured.

In commercial production of rasionuclide generators large quantities of tungsten are necessary. Accordingly, because of its high mass to volume ratio, tungsten metal is a preferred starting material. Unlike tungsten troxide, tungsten metal is converted to tungstate by dissolving the metal in an acidic solution, e.g., hydrofluoric acid and nitric acid. For example, tungsten metal and 48% hydrofluoric acid are placed in a Teflon beaker, whereupon concentrated nitric acid is added to the mixture to produce a clear solution. The solution is then evaporated to dryness under gentle heat to remove excess hydrofluoric acid. The resulting solid is then redissolved in base to produce the basic tungstate solution used in the present invention.

The pH of the acidic and basic solutions is selected to produce a final slurry or gel which remains acidic (pH 0 to 1) after the addition of the basic tungsten solution. It is preferable to slowly add the basic tungsten solution to the acid solution and not the acidic solution to the basic solution, to promote formation of zirconyl tungstate. Slow addition can be achieved, for example, by drop-wise addition. This is because of the tendency of the zirconyl cation to form zirconium hydroxide, which reaction competes with the reaction of zirconyl cation and tungsten, potentially reducing the amount of zirconyl tungstate formed. In addition, because a certain amount of zirconium hydroxide will tend to form in the bare of the combined solutions, a slight excess (preferably from 25% to 50%) of zirconium may be used to ensure formation of a zirconyl tungstate precipitate containing a 1:1 ratio of zirconium to total tungsten, thus compensating for the loss of some zirconyl to zirconium hydroxide. After precipitation, the slurry is adjusted to a pH of preferably about 2.8 to about 6.0, and most preferably about 4.3. Such pH controls enhance the elution efficiency of the generator of the present invention. Although the inventors do not wish to be bound by any theory, they believe that the pH control of the final precipitate improves the lattice structure of the gel matrix. A base such as sodium hydroxide (NaOH), the present redissolving of the zirconyl tungstate, may be used to adjust the pH of the precipitate. Any zirconium hydroxide which forms from the excess zirconyl cation used, as described above, may precipitate when the slurry is adjusted to a basic pH. This precipitate may remain associated with the matrix and absorb any solubilized tungsten released from the matrix which would otherwise contaminate the eluate.

The slurry is centrifuged and the precipitate is washed several times using deionized water or physiological saline and is filtered to remove any soluble tungsten (W-186 and W-188) not initially precipitated and, then, preferably, the precipitate is slowly air dried to remove excess liquid at room temperature. Alternatively, the precipitate may be oven dried, for example, at 100°C. The resulting glassy material which remains hydrated, is broken up, for example, using a spatula or by sonication to form the generator matrix material.

The commercial expedients of small volume and high efficiency for radionuclide generators require that particle size and shape be uniform to enhance the packing and elution characteristics of the generators. Unfortunately, the glassy nature of the precipitate makes it very difficult to break up and grind into uniform particles for column preparation. This is a significant practical problem. However, the inventors have discovered that uniform particles can be achieved more readily by addition of an inert tiller, for example an inorganic metal oxide, to the acidic zirconyl solution prior to reaction with the basic tungstate solution. The weight:volume ratio of filler to zirconyl solution is in the range of about 0.2% to about 5.0%, preferably about 1.0%. Examples of inert fillers include silica, quartz, alumina, magnesium, and diatomaceous earth.

A preferred purification protocol employing centrifugation and washing, rather than filtering, also improves the uniformity of particle size and elution efficiency. The preferred protocol requires at least three centrifugation/washing/decanting steps sequentially with water, a polar organic solvent miscible with water, and an organic solvent miscible with the polar organic solvent. Adequate washing requires sufficient volumes of wash solution relative to mother liquid at least in a ratio of about 1:5, e.g., 4 ml of wash per 20 ml of mother liquid, preferably in a ratio of about 1:1. This protocol removes soluble salts as well as water from the precipitate that may cause undesirable association between particles. Also, in contrast to air or heat drying, the precipitate is ground more easily into uniform particles. Accordingly, addition of an inert filler may not be necessary. Examples of polar organic solvents useful in the preferred purification protocol include acetone, acetonitrile, ethanol, methanol, and methylethyl ketone. Preferably, the organic solvent is selected to have a low boiling point to accelerate drying of the precipitate. Examples of organic solvents include diethylether, petroleum ether, hexane, pentane, methylene chloride, chloroform, tetrahydrofurane, di-isopropyl ether, and benzene.

The matrix material may be transferred to an empty container for eluting and harvesting of the daughter product Re-188. Suitable containers may include, for example, a glass column such as those used in standard chromatography which is then encased in a "shell" including appropriate lead shielding, associated plumbing and a reservoir of eluant, to form a generator assembly. Alternatively, a separate, sterile reservoir may be supplied for each series of elutions. It is desirable, but not essential, to keep the matrix hydrated at all times. Periodically, the daughter Re-188 is conveniently eluted from the column using eluant solutions, such as water or saline, for example sodium chloride (NaCl) or sodium sulfate ($Na_2SO_4$). Physiological saline, preferably with a molarity of 0.15, is a preferred eluant solution.

Performance of the rhenium generator of the present invention may be expressed as elution efficiency. Elution efficiency may be calculated by measuring the amount of radioactivity of Re-188 present in the eluant divided by the amount of radioactivity of Re-188 originally present on the generator column, immediately prior to elution. The radioactivity of the Re-188 may be determined using standard instruments for measuring radioactivity including gamma ray spectrophotometers such as germanium detectors and sodium iodide scintillation spectrophotometers, which are capable of measuring low levels of radioactivity, or dose calibrators that can measure high levels of radioactivity. In the present invention, since the generator consists of a small column, the entire column may be placed in a dose calibrator to directly measure the radioactivity of Re-188 on the column before elution, and by subtracting from this value the amount of radioactivity of Re-188 on the column after elution, the amount of radioactivity of the Re-188 present in the eluant may be determined. This procedure provides a close approximation of the Re-188 present in the eluant because, at the appropriate setting on the dose calibrator, the radioactivity measured on the column may be attributed to Re-188. Elution efficiencies are typically measured after approximately 3 to 10 Re-188 half-lives. Elution efficiencies of Re-188 as high as 55% - 65% have been obtained using the generators of the present invention, with concentrations of Re-188 in the eluant of up to 4 mCi/ml and higher, determined immediately after elution and typically after 3 or 4 halt-lives.

The radiochemical purity of Re-188 may be assessed using ion exchange, reversed phase high-performance liquid chromatography (HPLC) or scintillator chromatography using nonradioactive perrhenate as a standard.

While zirconyl tungstate ($ZrOWO_4$), or $ZrOWO_4 \cdot XH_2O$ when hydrated, is the preferred compound for forming the matrices used to generate Re-188 in the present invention, other suitable matrices include tungstate compounds containing hafnium, titanium, cerium, iron, tin and barium, and mixtures of these compound.

During the elution process, a certain amount of W-188 and W-186 in the eluant may be released from the matrix, for example, in the form of small particles of the zirconyl tungstate matrix, causing contamination of the Re-188. A porous glass or plastic structure, such as a fritted glass disc used in chromatography columns, may be used to retain some of these particles to prevent entry of tungsten into the eluate. However, the amount of W-188 released from the column is relatively low using the process of this invention (as low as 0.01%). This is because, in the present invention, a large traction of the generator matrix would have to dissolve before a substantial fraction of the W-188 contained in it is released. Using the generator of the present invention, Re-188 may be eluted with less than 0.01% (of the total W-188 present on the column) breakthrough of W-188. Moreover, the level of W-188 present in the eluate may be reduced by several orders of magnitude using a substrate which is capable of adsorbing tungsten including W-188, such as an alumina column or zirconium hydroxide bed, to purify the solution eluted from the zirconyl tungstate matrix. Thus, the generator system of the present invention may include a second elutable container, such as chromatographic column enclosing a second matrix containing such a tungsten-specific matrix, for removing any released W-188, in addition to the container enclosing the generator matrix. Alternatively, the substrate which is capable of adsorbing tungsten may be incorporated into the generator column, for example below the zirconyl tungstate matrix, so that the eluant passes through the substrate after first flowing through the zirconyl tungstate matrix. An additional advantage of the use of the tungsten-adsorbing substrate is that the loss of small particles of matrix may be minimized, which in turn decreases the amount of eluted fluid containing such contamination particles which must be disposed of.

W-188/Re-188 generator devices made according to the present invention are quite compact and may be made using small masses of generator matrix. Since the W-188 can be produced at a specific activity of approximately 1 Curie (Ci)/gram or higher by neutron capture, it is apparent that small (Curie size) generator columns containing volumes as low as 5 ml may be constructed using this process.

Although the generators of the present invention offer significant advantages over the prior art, the cost of the W/Re generators is significantly higher than most prior art radionuclide generators, e.g., molybdenum/technetium generators, primarily because of the more expensive W-186 starting material. Accordingly, there is significant economic motivation to develop a method for recycling and extraction of the tungsten-186 from generators that have already been fully exhausted by elution. Although no longer clinically useful, exhausted generators still could contain large amounts of radioactivity, e.g., as much as 200 mCi of W-188, even after a few monts since the half-life of W-188 is about 70 days. Thus, the present invention is also directed to methods for extracting the tungsten content from the exhausted zirconium tungstate gel. Once recovered, W-186 can be irradiated to form W-188 for use in preparing new generators. The basic recycling process comprises dissolving the zirconium tungstate gel or matrix, separating the zirconium from the tungsten, and isolating the tungsten. Dissolution of the gel can be achieved by reating with strong acid solution, for example concentrated hydrochloric acid. The separation step can be achieved, for example, by addition of a strong base to the gel solution which will dissolve the tungstate fraction of the gel while zirconium will precipitate out as zirconium oxide. Isolation can be accomplished, for example, by filtering the resulting solution, washing the precipitate, and recovering the filtrate containing tungsten. The tungsten is isolated in the filtrate and the volume is reduced. The tungsten can be recovered in one of two ways. First, the concentrated tungstate solution is heated causing evaporation of the water and forming tungsten trioxide; then by using a hydrogen reduction step under high pressure and temperature, the tungsten trioxide is converted to tungsten metal. This method, which is easily applied to enriched or non-radioactive tungsten, could be applicabel to radioactive samples. A second approach comprises using the technique of electroplating. Using the following equation, a current of a predetermined intensity is passed through the concentrated tungstate solution, producing the deposition of tungsten metal at the cathode:

$$WO_4^{2-} + 4H_2O + 6e^- \rightleftarrows W + 8\,OH^- \qquad E^0 = -1.05V$$

The cathode can be conveniently formed from W-186 enriched wire or foil, the mass of which would then be increased by the amount of tungsten metal deposited on the cathode. For example, a wire of 0.25 mm in diameter and 20 cm in length would correspond to 200 mg of tungsten metal. The wire could then be prepared for a new cycle of irradiation to W-188.

The following Examples are presented to illustrate the advantages of the present invention and to assist one of ordinary skill in making and using the same. The Examples are not intended in any way to otherwise limit the scope of the disclosure or the protection granted by Letters Patent hereon.

EXAMPLE 1

Preparation of W-188

116 mg of isotopically enriched W-186 in the form of tungsten trioxide (Oak Ridge National Laboratories, Oak Ridge, TN) was neutron-irradiated at $3 \times 10^{14}$ neutrons/cm²/sec for four to five months using the Missouri University Research Reactor to produce approximately 60 mCi of W-188 in the 116 mg of tungsten trioxide.

Preparation of Zirconyl-Tungstate Precipitate

116 mg of tungsten trioxide, irradiated as described above after decaying four months to a total activity of approximately 14 mCi, was added to 134 mg of non-irradiated $WO_3$ to equal 250 mg of $WO_3$, which was dissolved in 5 ml of 5 M sodium hydroxide (NaOH) heated to 60°C. The resulting basic tungsten trioxide solution was allowed to cool to room temperature, and then slowly added to an acidic solution, approximately 7.5 ml of 4 M HCl (to provide an excess of acid in the final solution) and containing 0.45 grams of zirconium nitrate (approximately 25% excess zirconium to ensure precipitation). The final slurry had a pH of 0 to 1 after addition of the basic tungsten solution. A white zirconyl tungstate precipitate formed immediately upon stirring and was neutralized using NaOH to a pH in the range of approximately 5 to 7 to prevent redissolving of the precipitate. The precipitate was then isolated by filtration and washed with physiological saline (0.9% NaCl) and then slowly dried at 25°C. The resulting glassy-white material was broken up using a spatula and transferred to an empty glass column (approximate volume 5-7 ml), containing a fritted glass disc (Mallinckrodt, Inc., St. Louis, MO). In the column, the matrix was again washed using physiological saline to release and remove any small particles from the matrix, to prevent entry of tungsten into the eluate, i.e., to reduce breakthrough. Water may also be used to wash the column.

W-188 was found to comprise less than 0.003% of the generator matrix, as determined from the radioactivity (mCi) of W-188 as measured using a dose calibrator.

Rhenium Generator

The above generator matrix column was fitted within a conventional housing of lead shielding, also containing an eluant reservoir and associated plumbing. Prior to the initial elution, the column was grain washed using approximately 500 ml of physiological saline, to further reduce breakthrough from the matrix. Re-188 was then allowed to generate from approximately 7 mCi of W-188 within the column for 1 to 2 days after which the Re-188 was eluted using 5 to 10 ml of physiological saline. 4 mCi of Re-188 in the form of $ReO_4^-$ was obtained in the initial elution using the above procedure. Subsequently, 2 to 4 mCi of Re-188 was obtained from each elution over a three-week time period, using a single elution every other day.

The concentration profile (mCi/ml of eluate) of Re-188 obtained from the generator may be determined by measuring the radioactivity of the Re-188 obtained in a given volume of eluate for several solutions. These measurements may be compared to the total amount of radioactivity of the Re-188 generated on the column to determine at what time point in elution the highest activity of Re-188 is obtained. In addition, the specific activity of Re-188 obtained from the column may be measured by determining the total radioactivity of Re-188 present in the eluate from the column.

### Elution Purity

To determine the amount of W-188 released from the matrix, eluates obtained as described above were analyzed using a multichannel analyzer (Nuclear Data, Inc., Schaumburg, IL) which showed that the W-188 content of the Re-188 solutions was between .008-.01% of total activity on the column. The amount of breakthrough of W-188 was further reduced by passage over an alumina column or hydrous zirconium oxide adsorbent bed. No other significant radionuclide impurities were present.

Nonradioactive chemical impurities such as metals present in the eluates may be examined using atomic absorption of elutions.

### Radiochemical Purity of Re-188

Perrhenate was the only material detected on an HPLC column after elution of the W-188/Re-188 generators described above.

### Generator Performance

Generator performance was measured in terms of elution efficiency as described above using a sodium iodide spectrophotometer and a dose calibrator. Generator efficiencies were found to be from 55% to 65%.

The generator described herein may be made to hold from several hundred milligrams up to 1 gram of target tungsten trioxide to provide daughter Re-188 yields of several hundred millicuries.

### Reusability of Generator

The generator prepared as described above was eluted regularly every other day for 2 to 3 weeks, producing an average elution yield of 59%. Other W-188/Re-188 generators made according to the process described herein were found to give similarly consistent high yields over several months'time. This demonstrates that generators as described herein have a useful lifetime for production of Re-188.

The present invention avoids the need to use a high specific activity parent radionuclide produced from fission. Since W-188 is unlikely to be available carrier-free in the foreseeable future, the present invention, which uses W-188 produced from W-186, provides a convenient means for obtaining carrier-free Re-188 in useful quantities without requiring large columns which are difficult to shield and which require large volumes of eluate to obtain acceptable amounts of product. Previous smaller Re-188 generators could not hold enough adsorbed W-188 and attempts to increase the generator activity reduced the elution yield of Re-188 and increased release of W-188 from the column.

The Re-188 produced by the generator device described herein may be conjugated to antibodies, for example, those that recognize tumor-associated antigens, for radiotherapy or diagnostic purposes. In addition, the relatively long half-life of W-188 (69.4 days), and ready transportability of the generator matrix in columns or other containing devices, facilitates commercial supply and storage of the generator matrices produced according to the present invention.

### EXAMPLE 2

### Sample Preparation and Irradiation

Two 250 mg preparations of isotopically enriched W-186 as tungsten trioxide ($WO_3$) 96% enriched (1.07 mmole) were introduced into separate T-21 quartz vials. The quartz vials were sealed with a torch under vacuum to a length of about 1.5 inches. Each seal was checked visually and with a needle probe. Each of the 250 mg preparations were then concurrently subject to the following procedure.

The sample was then irradiated in a nuclear reactor. After irradiation, the irradiated vial was returned in a numbered lead pig. After a minimum 15 days decay, the activity in the quartz vial was measured, using a dose calibrator at 217, 516, and 522 settings.

About 20 mL aqua regia was prepared by mixing about 15 mL ultrapure HCl and about 5 mL ultrapure nitric acid in a 50 mL sterile first polypropylene cup. The quartz vial was placed in a 50 mL sterile second polypropylene cup pierced with at least 10 holes from an 18G needle. The first polypropylene cup was placed into the second polypropylene cup and left in contact for 24 hours. About 20 mL of sterile water for injection was placed into a third 50 mL sterile polypropylene cup, and the second polypropylene cup was rinsed into the third cup. As much sterile water as necessary was used to rinse the quartz vial so that the wash solution showed a pH greater than pH 4 by using a pH strip indicator. The quartz vial was dried on sterile gauze and then placed in a 50 mL sterile fourth polypropylene cup that contained about 20 mL of 95% ethanol. The quartz vial was dried on sterile gauze.

## Dissolution of the Target

The quartz vial was introduced into a radioisotope glove box. Using a scoring tool and vial breaker that have been cleaned with ethanol, the quartz vial was opened in the glove box. The irradiated powder and the opened vial were placed into a sterile, pyrogen free 100 mL beaker equipped with a sterile, pyrogen free stirbar. Using a 3cc sterile disposable syringe, 2.0 mL sodium hydroxide 10 M was withdrawn and added to the beaker containing the irradiated powder and the opened vial. Using a 10cc sterile disposable syringe, 8.0 mL of sterile water was withdrawn for injection and added to the beaker containing the irradiated powder and the opened vial. The beaker was placed on a hot plate stirrer and heated gently on setting 1.6 (about 85°C) while stirring for at least 15 minutes until the tungsten trioxide had dissolved. The beaker was then placed on another stirrer and stirred. Using a 3cc sterile disposable syringe without a needle, 3.0 mL ultrapure HCl was withdrawn and then added slowly to the beaker over a 5 minute period. A gray precipitate was formed.

Using a sterile plastic pipette, a total of 1 mL of hydrogen peroxide was added over a period of 1 hour, starting with 0.4 mL and followed by three times 0.2 mL every 15 minutes. Using a 3cc sterile disposable syringe, about 1.4 mL of sodium hydroxide 10 M then was added in a drop-wise manner as required to obtain a clear solution. Using a 10cc sterile disposable syringe, 5.0 mL of sterile water for injection was withdrawn. Using beta shielded tongs, the pieces of the quartz vial from the beaker containing the radioactive solution and drain were removed. The quartz fragments were rinsed with the water contained in the 10cc syringe described above. The quartz fragments were placed in a 10 mL vial containing 2.5 mL sodium hydroxide 10 M and 2.5 mL sterile water, and set aside. The beaker was placed on a hot plate stirrer and heated gently to completely evaporate the solution while stirring. The heater setting was 75°C for 25 minutes. The beaker was removed from the hot plate stirrer and cooled at room temperature for 5 minutes. Using a 10cc sterile disposable syringe, 8.0 mL sterile water for injection was withdrawn, and added to the beaker. The beaker was placed on a stirrer and stirred for complete dissolution.

## Preparation of Zirconyl Nitrate Solution

570 mg zirconyl nitrate (2.14 mmole) was placed into a 100 mL sterile, pyrogen free beaker equipped with a sterile, pyrogen free stirbar. The beaker was placed in the radioisotope glove box. Using a 3cc sterile disposable syringe, 2.2 mL ultrapure HCl was withdrawn, and added to the beaker containing the zirconyl nitrate. Using a 10cc sterile disposable syringe, 4.4 mL sterile water for injection was withdrawn, and added to the beaker containing the zirconyl nitrate. The beaker was placed on the hot plate stirrer; heated gently on setting 1.0 (about 55°C) while stirring until completely dissolved. The beaker was placed on another room temperature stirrer and stirred.

## Precipitation of Zr-W gel

A sterile disposable 10cc syringe was prepared for precipitation by removing its barrel and placing at the luer lock a sterile, disposable three-way valve. A ringstand was used to hold this syringe over the assembly made of the stirrer and the beaker containing the zirconyl nitrate solution. A combination pH electrode was calibrated using a single point calibration at pH 4.0. The electrode was rinsed with sterile water for injection. The radioactive solution of the target was transferred into the sterile 10cc syringe using a beta shielded sterile disposable pipette. The two-way valve was rotated to allow drop-wise addition of radioactive solution. Using a 3cc sterile disposable syringe, 3 mL of sterile water for injection was withdrawn, and 1.5 mL added to the beaker that contained the radioactive tungsten solution. The rinse was transferred to the 10cc syringe and added to the precipitated gel. A second rinse was made under identical conditions using the second 1.5 mL volume of water. The pH electrode was immersed into the beaker containing the gel solution. The pH of the gel solution was adjusted to 4.0 - 4.3 with about 2.8 mL of sodium hydroxide 10 M. Using a 3cc sterile disposable syringe, 3 mL of sterile water for injection was withdrawn. The electrode was removed and washed with two times 1.5 mL water. The precipitated gel was stirred for 1 hour at room temperature. A filter membrane was placed in the filtration apparatus, and the filtration apparatus placed on a sterile pyrogen free 500 mL filter flask. Sterile, pyrogen free reinforced plastic tubing was connected between the filter flask and another sterile flask which was connected to a sterile, vacuum hand pump. Using a shielded, sterile disposable pipette, the gel solution was placed into the filtration apparatus. Using the vacuum hand pump, the gel was filtered. The beaker that contained the gel was rinsed two times with about 20 mL sterile water for injection, and filtered. The gel was washed two times with about 100 mL sterile water for injection by pouring it on the side of the filter holder to avoid breaking the precipitate. The filtrate and washes were collected and an aliquot was withdrawn for calculation of precipitation yields.

## Gel drying

The gel was dried under reduced pressure of 3.0 inches of water ± 0.5 inches, for at least 18 hours in the glove box at room temperature.

The dried gel was placed into a 10cc sterile, pyrogen free vial by dumping the gel onto a sterile, pyrogen free funnel. It is important to do this step very carefully as the particulates are highly radioactive. A piece of parafilm was wrapped around a beta shielded spatula inside the vial containing the dried gel. The gel was ground until small particulates were obtained. 250 mg hydrous zirconium oxide was weighed into two sterile, pyrogen free generator columns, using a calibrated microbalance. The generator column A was prepared with the funnel used previously. About two thirds of the gel required was poured into the funnel to pack the Zr-W generator column A. The funnel was then placed on top of generator column B and the other third of the gel was poured the same way onto generator column B. The tops of the columns were sealed using a sterile rubber septa, aluminum seals and crimpers. The columns were then wiped out with adsorbing paper to remove any radioactive particles that would adsorb onto the columns. The columns were then placed in a plastic bag, then in a lead container of appropriate size.

While the present invention has been described in conjunction with the preferred embodiments, one of ordinary skill after reading the foregoing specification will be able to effect various changes, substitutions of equivalents and alterations to the methods and compositions set forth herein. It is therefore intended that the protection granted by Letters Patent hereon be limited only by the appended claims and equivalents thereof.

## Claims

1. A process for preparing a radionuclide generator useful for producing Re-188, comprising the steps of:
(a) reacting a basic tungstate solution comprising W-188 with an acidic solution comprising zirconyl ion to form a zirconyl tungstate precipitate containing W-188 thereby forming a final gel or slurry which remains acidic,
(b) optionally adjusting the pH of said precipitate from about 2.8 to about 6; and,
(c) disposing said precipitate in an elutable container.

2. A process according to Claim 1, wherein said pH of the precipitate is adjusted to about 4.3.

3. A process according to Claim 1 or 2, wherein said basic tungstate solution is added to said acidic solution in a drop-wise manner.

4. A process according to Claim 1, 2 or 3, wherein the pH of said precipitate is adjusted by addition of base.

5. A process according to any of claims 1 to 4, wich further comprises the additional step between steps (b) and (c) of:
sequential washing, centrifugation, and decating with water, then a polar organic solvent miscible with water, and then an organic solvent miscible with said polar organic solvent.

6. A process according to Claim 5, wherein said polar organic solvent is selected from the group consisting of acetone, acetonitrile, ethanol, methanol, and methylethyl ketone.

7. A process according to Claim 5, wherein said organic solvent has a low boiling point to accelerate drying of said precipitate.

8. A process according to Claim 5, wherein said organic solvent is selected from the group consisting of diethyl ether, petroleum ether, hexane, pentane, methylene chloride, chloroform, tetrahydrofuran, di-isopropyl ether, and benzene.

9. A process according to any of the preceding claims wherein said acidic solution further comprises an inert filler.

10. A process according to Claim 9, wherein said filler is an inorganic metal oxide.

11. A process according to Claim 9, wherein said filler is selected from the group consisting of silica, quartz, alumina, magnesium oxide, and diatomaceous earth.

12. A radionuclide generator for producing Re-188, comprising a substantially insoluble matrix of zirconyl tungstate prepared according to the process of any of the preceding claims.

**Patentansprüche**

1. Verfahren zur Herstellung eines Rasionuklidgenerators zur Erzeugung von 188$^{Re}$, das folgende Stufen umfaßt:

a) Umsetzung einer basischen, W$^{188}$ enthaltenden Wolframatlösung mit einer sauren, Zirkonylionen enthaltenden Lösung zur Bildung eines W$^{188}$ enthaltenen Zirkonylwolframat-Niederschlags und auf diese Weise zur Bildung eines Gels oder einer Suspension als Endprodukt, das sauer bleibt,

b) gegebenenfalls Einstellung des pH's des Niederschlags auf ca. 2,8 bis ca. 6 und

c) Überführung des Niederschlags in ein eluierbares Gefäß.

2. Verfahren nach Anspruch 1, worin der pH des Nierderschlags auf ca. 4,3 eingestellt wird.

3. Verfahren nach Anspruch 1 oder 2, worin die basische Wolframatlösung der sauren Lösug zugetropft wird.

4. Verfahren nach Anspruch 1, 2 oder 3, worin der pH des Niederschlags durch Zugabe einer Base eingestellt wird.

5. Verfahren nach einem der Ansprüche 1 bis 4, das außerdem zwischen den stufen b) und c) noch eine zusätzliche Stufe umfaßt:

nachfolgendes Waschen, Zentrifugieren und Dekantieren mit Wasser, danach mit einem mit Wasser mischbaren polaren organischen Lösungsmittel und dann mit einem mit dem polaren organischen Lösungsmittel mischbaren organischen Lösungsmittel.

6. Verfahren nach Anspruch 5, worin das polare organische Lösungsmittel ausgewählt ist aus der Gruppe Aceton, Acetonitril, Ethanol, Methanol und Methylethylketon.

7. Verfahfren nach Anspruch 5, worin das organische Lösungsmittel einen niedrigen Kochpunkt aufweist, um die Trocknung des Niederschlags zu beschleunigen.

8. Verfahren nach Anspruch 5, worin das organische Lösungsmittel ausgewählt ist aus der Gruppe Diethylether, Petrol-ehter, Hexan, Pentan, Methylenchlorid, Chloroform, Tetrahydrofuran, Diisopropylether und Benzol.

9. Verfahren nach einem der vorangegangenen Ansoprüche, worin die saure Lösung außerdem noch einen inerten Füller enthält.

10. Verfahren nach Anspruch 9, worin der Füller ein anorganisches Metalloxid ist.

11. Verfahren nach Anspruch 9, worin der Füller ausgewählt ist aus der Gruppes Kieselerde, Quarz, Tonerde, Magnesiumoxid und Diatomeenerde.

12. Radionuklidgenerator zur Herstellung von 188$^{Ra}$, der eine im wesentlichen unlösliche Masse aus Zirkonylwolframat umfaßt, hergestellt nach dem Verfahren gemäß einem der vorangegangenen Ansprüche.

**Revendications**

1. Procédé pour préparer un générateur de radionucléides permettant de produire du Re-188, comportant les étapes qui consistent à :

a) faire réagir une solution basic de tungstate comportant du W-188 avec une solution acide comportant un ion zirconyle pour former un précipité de tungstate de zirconyle contenant du W-188 afin de former un gel ou une boue qui reste acide,

b) ajuster, à titre facultatif, le pH dudit précipité entre 2,8 et environ 6, and

c) disposer ledit précipité dans un conteneur d'élusion.

2. Procédé selon la revendication 1, dans lequel ledit pH du précipité est ajusté à environ 4,3.

3. Procédé selon la revendication 1 ou 2 dans lequel ladite solution basic de tungstate est ajoutée à ladite solution acide goutte à goutte.

4. Procédé selon la revendication 1, 2 ou 3 dans lequel le pH dudit précipité est ajusté par addition d'une base.

5. Procédé selon l'une quelconque des revendications 1 à 4 qui comporte en outre une étape aditionnelle entre les étapes (b) et (c) qui consiste en :

un lavage séquentiel, une centrifugation et une décantation avec de l'eau, alors l'addition d'un solvant organique polaire miscible avec l'eau et alors d'un solvant organique miscible avec ledit solvant organique polaire.

6. Procédé selon la revendication 5, dans lequel ledit solvant organique polaire est choisi dans le groupe qui comporte l'acétone, l'acétonitrile, l'éthanol, le méthanol, et la méthyléthylcétone.

7. Procédé selon la revendication 5, dans lequel ledit solvant organique a un point d'ébullition faible pour accélerer le séchage dudit précipité.

8. Procédé selon la revendication 5 dans lequel ledit solvant organique est choisi dans le groupe qui comporte l'éther diéthylique, l'éther de pétrole, l'hexane, le pentane, le chlorure de méthylène, le chloroforme, la tétrahydrofuranne, l'éther di-hysopropylique et le benzène.

9. Procédé selon l'une quelconque des revendications précédentes dans lequel ladite solution acide comporte en outre une charge inerte.

10. Procédé selon la revendication 9 dans lequel ladite charge est un oxyde métallique inorganique.

11. Procédé selon la revendication 9, dans lequel ladite charge est choisie dans le groupe qui comporte la silice, le quartz, l'alumine, l'oxyde de magnésium et la terre de diatomées.

12. Générateur de radionucléides pour produire du Re-188 comportant une matrice sensiblement insoluble de tungstate de zirconyle préparé selon le procédé de l'une quelconque des revendications précédentes.